(19)

Europäisches Patentamt

European Patent Office

Office européen des brevets

(11) **EP 4 122 471 A1**

(12) **EUROPEAN PATENT APPLICATION**
published in accordance with Art. 153(4) EPC

(43) Date of publication:
**25.01.2023 Bulletin 2023/04**

(21) Application number: **21750936.3**

(22) Date of filing: **05.02.2021**

(51) International Patent Classification (IPC):
*A61K 31/7088* (2006.01)      *A61K 45/00* (2006.01)
*A61P 27/02* (2006.01)      *C12N 15/115* (2010.01)

(52) Cooperative Patent Classification (CPC):
**A61K 31/7088; A61K 45/00; A61P 27/02;**
**C12N 15/115**

(86) International application number:
**PCT/JP2021/004215**

(87) International publication number:
**WO 2021/157681 (12.08.2021 Gazette 2021/32)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **06.02.2020  US 202062970842 P**

(71) Applicant: **Ribomic Inc**
**Tokyo 108-0071 (JP)**

(72) Inventors:
• **NAKAMURA, Yoshikazu**
**Tokyo 108-0071 (JP)**
• **FUJIWARA, Masatoshi**
**Tokyo 108-0071 (JP)**
• **ALI, Yusuf**
**Berkeley, California 94704 (US)**
• **PEREIRA, Daniel**
**Berkeley, California 94704 (US)**

(74) Representative: **J A Kemp LLP**
**80 Turnmill Street**
**London EC1M 5QU (GB)**

Remarks:
The complete document including Reference
Table(s) and the Sequence Listing(s) can be
downloaded from the EPO website

(54) **THERAPEUTIC AGENT FOR SUBRETINAL HYPERREFLECTIVE MATERIAL OR RETINAL DISORDERS ACCOMPANYING SUBRETINAL HYPERREFLECTIVE MATERIAL**

(57)    An effective therapeutic agent for subretinal hyperreflective material or retinal diseases accompanying subretinal hyperreflective material is provided by the present invention. Specifically, a therapeutic agent for subretinal hyperreflective material or a retinal disease accompanying subretinal hyperreflective material, containing an aptamer that binds to FGF2 or a salt thereof is provided.

[Fig. 1]

EP 4 122 471 A1

**Description**

[Technical Field]

The present invention relates to a therapeutic agent for subretinal hyperreflective material or retinal diseases accompanying subretinal hyperreflective material, containing an aptamer that binds to FGF2 or a salt thereof.

[Background Art]

Age-related macular degeneration (AMD) is a disease that causes disorder in the macula in the center of the retina and leads to blindness when it is exacerbated. About 1% of people over the age of 50 develop age-related macular degeneration, and it is the number one cause of blindness in Europe and the United States. The number of age-related macular degeneration patients worldwide is expected to reach about 200 million in 2020.

Age-related macular degeneration is roughly divided into atrophic type (dry type) and exudative type (wet type). Atrophic type age-related macular degeneration is a disease in which retinal pigment epithelial cells become inflamed due to aging, and the eyesight is distorted due to the loss of the retinal pigment epithelial cells together with the photoreceptor cells above them. Until now, there is no effective therapeutic drug for atrophic type age-related macular degeneration, and prevention by improving lifestyle and the like is the main measure. On the other hand, exudative type age-related macular degeneration is a disease in which abnormal neovascularization occurs from the choroid due to deterioration of the function of retinal pigment epithelial cells due to aging or damage to the retinal pigment epithelial cells, and the function of photoreceptor cells is disrupted due to bleeding from the new blood vessels or leakage of blood components.

The treatment of exudative type age-related macular degeneration has made drastic progress in the last 15 years, and several therapeutic agents have been placed on the market. For example, VEGF inhibitors that inhibit the formation of abnormal new blood vessels in the retina have been used as therapeutic agents for age-related macular degeneration. It has been reported that these VEGF inhibitors have a therapeutic effect on about half of patients with exudative type age-related macular degeneration, but are not effective for 30 to 50% of patients (Non Patent Literature 1). It has also been reported that even in patients who showed confirmed therapeutic effects, the eyesight begins to deteriorate several years after the start of treatment (Non Patent Literature 2).

As one of the causes of poor prognosis in patients with exudative type age-related macular degeneration, subretinal hyperreflective material (SHRM) caused by exudative type age-related macular degeneration is attracting attention (Non Patent Literature 3). When this SHRM is present, the therapeutic effect is considered to be low even if the abnormal new blood vessels are removed. In particular, SHRM cannot be removed by an anti-VEGF drug, and therefore, it is considered that the therapeutic effect of the anti-VEGF drug on exudative type age-related macular degeneration is limited (Non Patent Literature 4).

It has also been reported that this SHRM occurs not only in age-related macular degeneration but also in various other retinal diseases. For example, SHRM can be confirmed in the OCT images of the retina of patients with vitreoretinal lymphoma (Non Patent Literature 5); BEST disease (Non Patent Literature 6); retinal vitreous interface syndrome (Non Patent Literature 7); central serous chorioretinopathy (Non Patent Literature 8); or myotonic dystrophy (Non Patent Literature 9), and it has been reported that this is related to the therapeutic effect and poor prognosis of respective retinal diseases.

That is, the elimination of this SHRM is considered to be important for the treatment of many retinal diseases accompanying SHRM and the improvement of poor prognosis thereof.

On the other hand, no therapeutic agent for retinal diseases that can eliminate SHRM has been reported yet. Therefore, there is a demand for an effective therapeutic agent for subretinal hyperreflective material or retinal diseases accompanying subretinal hyperreflective material. Furthermore, there is no report that FGF2 inhibitors were also effective for subretinal hyperreflective material or retinal diseases accompanying subretinal hyperreflective material. In the meantime, the Applicant is intensively developing a pharmaceutical product containing an aptamer for FGF2 as an active ingredient.

[Citation List]

[Non Patent Literature]

[NPL 1]
Brown DM et al., N Engl J Med. 2006 Oct 5; 355(14):1432-44

[NPL 2]
Bhisitkul RB et al., Ophthalmology. 2016 Jun; 123(6):1269-77
[NPL 3]
Alex S. Willoughby, BS et al., Graefes Arch Clin Exp Ophthalmol (2018) 256:2089-2096
[NPL 4]
Ebenezer Daniel et al., Ophthalmology. 2014 March; 121(3): 656-666.
[NPL 5]
Cicinelli, Maria Vittoria et al., Retin Cases Brief Rep. 2019 doi: 10.1097/ICB.0000000000000952. [Epub ahead of print]
[NPL 6]
Daniela C. Ferrara et al., Graefes Arch Clin Exp Ophthalmol. 2010 October; 248(10): 1377-1386
[NPL 7]
Sarra Gattoussi et al., Acta Ophthalmol. 2019: 97; 364-371
[NPL 8]
Niroj K Sahoo et al., Indian Journal of Ophtalmology vol.68, Issue 1, 126-129
[NPL 9]
A Ozkaya et al., J Fr Ophtalmol. 2018, 41:21-29

[Summary of Invention]

[Technical Problem]

Therefore, an object of the present invention is to provide a therapeutic agent effective for subretinal hyperreflective material or retinal disease age-related macular degeneration accompanying subretinal hyperreflective material, each of which relates to poor prognosis of retinal disease patients.

[Solution to Problem]

The present inventors have conducted intensive studies in an attempt to achieve the aforementioned object and found in the development of therapeutic agents containing an FGF2 aptamer as an active ingredient that the FGF2 aptamer is effective in eliminating subretinal hyperreflective material (SHRM). No pharmaceutical product has been previously reported which is based on a mechanism in which SHRM is eliminated by inhibiting the function of FGF2 and retinal diseases are treated thereby.

The present inventors have further conducted intensive studies and finally completed the present invention.

Accordingly, the present invention provides the following.

[1] A therapeutic agent for subretinal hyperreflective material or a retinal disease accompanying subretinal hyperreflective material, comprising an aptamer that binds to FGF2 or a salt thereof.

[2] The therapeutic agent of [1], wherein the aforementioned aptamer is an aptamer comprising a nucleotide sequence represented by the following formula (1)

$$N^1GGAN^2ACUAGGGCN^3UUAAN^4GUN^5ACCAGUGUN^6 \qquad (1)$$

wherein $N^1$ and $N^6$ are each independently any 0 to several bases, and $N^2$, $N^3$, $N^4$ and $N^5$ are independently any one base, provided that uracil is optionally thymine: and is the following (a) or (b):

 (a) an aptamer wherein, in the nucleotides contained in the aptamer,

  (i) the 2'-position of the ribose of each pyrimidine nucleotide is a fluorine atom,
  (ii) the 2'-position of the ribose of each purine nucleotide is a hydroxy group;

 (b) the aptamer of (a), wherein

  (i) the fluorine atom at the 2'-position of the ribose of each pyrimidine nucleotide is independently not replaced, or replaced by an atom or group selected from the group consisting of a hydrogen atom, a hydroxy group, and a methoxy group,
  (ii) the hydroxy group at the 2'-position of the ribose of each purine nucleotide is independently not replaced, or replaced by an atom or group selected from the group consisting of a hydrogen atom, a methoxy group,

and a fluorine atom.

[3] The therapeutic agent of [2], wherein the nucleotide sequence represented by the aforementioned formula (1) is a nucleotide sequence represented by the following formula (3):

$$N^1GGAUACUAGGGCAUUAAUGUUACCAGUGUAGUCN^{62} \qquad (3)$$

wherein $N^1$ and $N^{62}$ are each independently any 0 to several bases.

[4] The therapeutic agent of [3], wherein the nucleotide sequence represented by the aforementioned formula (3) is a nucleotide sequence shown in any of SEQ ID NO: 1, 3, 4, 5, 6, or 8.

[5] The therapeutic agent of [3], wherein the nucleotide sequence represented by the aforementioned formula (3) is a nucleotide sequence shown in SEQ ID NO: 3.

[6] The therapeutic agent of any one of [2] to [5], wherein the aforementioned aptamer is an aptamer represented by the following:

```
GL2-400TS-C6-
G(M)G(M)G(M)A(M)U(M)A(M)C(M)U(F)A(M)G(M)G(M)GC(M)A(M)U(M)U(F)A(
M)A(M)U(M)G(M)U(F)U(M)A(M)C(M)C(M)A(M)GU(F)GU(F)A(M)G(M)U(M)C(M
)C(M)C(M)-idT
```

wherein (M) is a methoxy group at the 2'-position of ribose in each nucleotide, (F) is a fluorine atom at the 2'-position of ribose in each nucleotide, GL2-400TS is a 2-branched TS type polyethylene glycol having a molecular weight of 40000, C6 is a $-(CH_2)_6-$ linker, and idT is an inverted dT.

[7] The therapeutic agent of any one of [1] to [6], wherein the retinal disease is age-related macular degeneration.

[8] The therapeutic agent of any one of [1] to [7], wherein the agent is administered in combination with an anti-VEGF drug.

[9] A method for treating subretinal hyperreflective material or a retinal disease accompanying subretinal hyperreflective material, comprising administering an effective amount of an aptamer that binds to FGF2 or a salt thereof to a patient thereof.

[10] An aptamer that binds to FGF2 or a salt thereof for treating subretinal hyperreflective material or a retinal disease accompanying subretinal hyperreflective material.

[11] Use of an aptamer that binds to FGF2 or a salt thereof for the production of a therapeutic agent for subretinal hyperreflective material or a retinal disease accompanying subretinal hyperreflective material.

[Advantageous Effects of Invention]

According to the present invention, an effective therapeutic agent for SHRM, for which a therapeutic drug has not existed so far, can be provided. SHRM becomes fibrotic when left as is and can cause a fibrous retinal disease around the retina. That is, according to the therapeutic agent of the present invention, it is possible to treat subretinal hyperreflective material and various retinal diseases accompanying subretinal hyperreflective material, and improvement of the prognosis of various retinal diseases accompanying subretinal hyperreflective material can be expected.

[Brief Description of Drawings]

[Fig. 1]

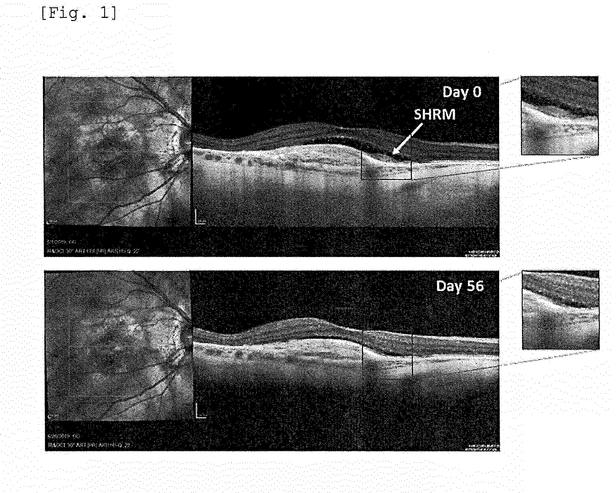

Fig. 1 shows a comparison of tissue evaluation by optical coherence tomography (OCT) scan, before and after treatment with the therapeutic agent of the present invention, in patients with age-related macular degeneration accompanying subretinal hyperreflective material.

[Description of Embodiments]

The present invention provides a therapeutic agent for subretinal hyperreflective material or retinal diseases accompanying subretinal hyperreflective material, containing an aptamer that binds to FGF2 (hereinafter also to be referred to as the aptamer of the present invention) or a salt thereof (hereinafter also to be referred to as the therapeutic agent of the present invention).

An aptamer refers to a nucleic acid molecule having a binding activity to a predetermined target molecule. The aptamer can inhibit the activity of a predetermined target molecule by binding to the target molecule. The aptamer of the present invention is an aptamer that binds to FGF2, preferably an aptamer that binds to FGF2 to inhibit the binding of FGF2 and an FGF receptor. The inhibition of the binding of FGF2 and an FGF receptor can be evaluated by a test utilizing, for example, the surface plasmon resonance method.

The aptamer of the present invention is not limited as long as it is an aptamer that binds to FGF2, preferably, an aptamer that binds to FGF2 to inhibit the binding of FGF2 and an FGF receptor. For example, it is the aptamer described in WO 2020/004607, specifically, an aptamer containing a nucleotide sequence represented by the following formula (1):

$$N^1 GGAN^2 ACUAGGGCN^3 UUAAN^4 GUN^5 ACCAGUGUN^6 \qquad (1)$$

provided that uracil is optionally thymine, and is the following (a) or (b):

(a) an aptamer wherein, in the nucleotides contained in the aptamer,

(i) the 2'-position of the ribose of each pyrimidine nucleotide is a fluorine atom,
(ii) the 2'-position of the ribose of each purine nucleotide is a hydroxy group;

(b) the aptamer of (a), wherein

(i) the fluorine atom at the 2'-position of the ribose of each pyrimidine nucleotide is independently not replaced, or replaced by an atom or group selected from the group consisting of a hydrogen atom, a hydroxy group and a methoxy group,
(ii) the hydroxy group at the 2'-position of the ribose of each purine nucleotide is independently not replaced, or replaced by an atom or group selected from the group consisting of a hydrogen atom, a methoxy group and a fluorine atom.

In the above-mentioned formula (1), $N^1$ and $N^6$ are each independently any 0 to several bases, and $N^2$, $N^3$, $N^4$ and $N^5$ are independently any one base. In the present specification, "base" means any of adenine (A), guanine (G), cytosine (C), uracil (U) or thymine (T) constituting a nucleic acid.

While the base number of $N^1$ is not particularly limited as long as an aptamer containing a nucleotide sequence represented by the formula (1) binds to FGF2, it may be, for example, 0 to about 10, 0 to 9, 0 to 8, 0 to 7, 0 to 6, 0 to 5, 0 to 4, 0 to 3, 0 to 2, and the like, preferably 0 to 2.

Similar to $N^1$, the base number of $N^6$ is not particularly limited. It may be, for example, 0 to about 10, 0 to 9, 0 to 8, 0 to 7, 0 to 6, 0 to 5, 0 to 4, 0 to 3, and the like, preferably 0 to 10, 3 to 9, or 5 to 8.

In a preferred embodiment, in the above-mentioned formula (1),

$N^1$ is G, GG, AG, C or a gap,
$N^2$ is A or U,
$N^3$ is G, C or A,
$N^4$ is G, C or U,
$N^5$ is G or U, and
$N^6$ is $UUCN^{61}$ or $AGUCN^{62}$ wherein $N^{61}$ and $N^{62}$ are each independently any 0 to several bases. Here, $N^1$ is a "gap" means that $N^1$ is absent in the formula (1), namely, $N^1$ is 0 base.

While the base number of $N^{61}$ is not particularly limited, it may be, for example, 0 to about 10, 0 to 7, 0 to 6, 0 to 5, 0 to 4, and the like, preferably 0 to 5, 1 to 5, or 2 to 4.

While the base number of $N^{62}$ is also not particularly limited, it may be, for example, 0 to about 10, 0 to 7, 0 to 5, 0 to 4, 0 to 3, and the like, preferably 0 to 5, 0 to 4, or 0 to 3.

In another preferred embodiment, in the above-mentioned formula (1),

$N^1$ is G, GG, AG or a gap,
$N^2$ is A or U,
$N^3$ is G or A,
$N^4$ is C or U,
$N^5$ is G or U,
$N^6$ is $UUCN^{61}$ or $AGUCN^{62}$ wherein $N^{61}$ and $N^{62}$ are as defined above.

In a preferred embodiment, the aptamer used in the present invention may contain a nucleotide sequence represented by the following formula (2) or (3):

$$GGGAAACUAGGGCGUUAACGUGACCAGUGUUUCN^{61} \qquad (2)$$

$$N^1GGAUACUAGGGCAUUAAUGUUACCAGUGUAGUCN^{62} \qquad (3)$$

wherein $N^1$, $N^{61}$ and $N^{62}$ are as defined above, more preferably, a nucleotide sequence represented by the formula (3).

In a preferred embodiment, the aptamer of the present invention contains a nucleotide sequence shown by SEQ ID NOs: 1 to 12. The nucleotide sequences shown in SEQ ID NOs: 1 to 12 are recited below (hereinafter A, G, C and U show that the base of nucleotide is adenine, guanine, cytosine or uracil, respectively) (provided that uracil is optionally thymine).

SEQ ID NO: 1:
GGGAUACUAGGGCAUUAAUGUUACCAGUGUAGUCUCGA
SEQ ID NO: 2:
GGGAAACUAGGGCGUUAACGUGACCAGUGUUUCUCGA
SEQ ID NO: 3:
GGGAUACUAGGGCAUUAAUGUUACCAGUGUAGUCCC
SEQ ID NO: 4:
GGAUACUAGGGCAUUAAUGUUACCAGUGUAGUCC
SEQ ID NO: 5:
GGGGAUACUAGGGCAUUAAUGUUACCAGUGUAGUCCCC
SEQ ID NO: 6:
AGGGAUACUAGGGCAUUAAUGUUACCAGUGUAGUCCC
SEQ ID NO: 7:
GGGAAACUAGGGCGUUAACGUGACCAGUGUUUCCC
SEQ ID NO: 8:
CGGAUACUAGGGCAUUAAUGUUACCAGUGUAGUCCG
SEQ ID NO: 9:
CCGAUACUAGGGCAUUAAUGUUACCAGUGUAGUCGG
SEQ ID NO: 10:
GGGAUACUAGGGCGUUAACGUUACCAGUGUAGUCCC
SEQ ID NO: 11:
GGGAUACUAGGGCCUUAAGGUUACCAGUGUAGUCCC
SEQ ID NO: 12:
GGGAUACUAGGGCAUUUAUGUUACCAGUGUAGUCCC

In one preferred embodiment, the aptamer of the present invention contains a nucleotide sequence shown in SEQ ID NO: 1, 3, 4, 5, 6, or 8, more preferably, a nucleotide sequence shown in SEQ ID NO: 3 (encompassed in the above-mentioned formula (3)).

In one embodiment, the aptamer of the present invention may contain, in any of the above-mentioned nucleotide sequences, a nucleotide sequence wherein 1 or several nucleotides are replaced, deleted, inserted or added, as long as the aptamer still binds to FGF2, and may be

(a) an aptamer wherein, in the nucleotides contained in the aptamer,

(i) the 2'-position of the ribose of each pyrimidine nucleotide is a fluorine atom,
(ii) the 2'-position of the ribose of each purine nucleotide is a hydroxy group;

(b) the aptamer of (a), wherein

(i) the fluorine atom at the 2'-position of the ribose of each pyrimidine nucleotide is independently not replaced, or replaced by an atom or group selected from the group consisting of a hydrogen atom, a hydroxy group and a methoxy group,
(ii) the hydroxy group at the 2'-position of the ribose of each purine nucleotide is independently not replaced, or replaced by an atom or group selected from the group consisting of a hydrogen atom, a methoxy group and a fluorine atom.

As used herein, the number of the above-mentioned nucleotides to be replaced, deleted, inserted or added is not particularly limited as long as the aptamer still binds to FGF2 even after the replacement, deletion, insertion or addition. It can be, for example, 1 to about 10, preferably 1 to 6, more preferably 1 to 5, further preferably 1 to 4, further preferably 1 to 3, most preferably 1 or 2. While the site of the nucleotide to be replaced, deleted, inserted or added is not particularly limited as long as the aptamer still binds to FGF2 even after the replacement, deletion, insertion or addition, at the sites specified to be one kind of nucleotide (i.e., A, G, C, or U) in the above-mentioned formula (1), (2) and (3), nucleotides are replaced, deleted, inserted or added at 1 to 3, preferably 1 or 2, more preferably 1, site. On the other hand, at a site where plural kinds of nucleotides may be present (i.e., $N^1$, $N^2$, $N^3$, $N^4$, $N^5$, $N^6$, $N^{61}$, or $N^{62}$) in the formulas (1), (2), and (3), more number of nucleotides (e.g., 1 to about 10, preferably 1 to 6, more preferably 1 to 5, further preferably 1 to 4) may be replaced, deleted, inserted, or added. For example, assuming that the nucleotide sequence shown in SEQ ID NO: 3 is the original sequence, SEQ ID NO: 1 is a sequence resulting from replacement of CC at the 3'-terminal of SEQ ID NO: 3 by UCGA, SEQ ID NO: 4 is a sequence resulting from deletion of one nucleotide each at both terminals of SEQ ID NO: 3, SEQ ID NO: 5 is a sequence resulting from addition of G to the 5'-terminal and C to the 3'-terminal of SEQ ID NO: 3, SEQ ID NO: 6 is a sequence resulting from addition of A to the 5'-terminal of SEQ ID NO: 3, SEQ ID NO: 8 is a sequence resulting from replacement of G at the 5'-terminal of SEQ ID NO: 3 with C and C at the 3'-terminal of SEQ ID NO: 3 with G, SEQ ID NO: 9 is a sequence resulting from replacement of GG at the 5'-terminal of SEQ ID NO: 3 by CC and CC at the 3'-terminal of SEQ ID NO: 3 with GG, SEQ ID NO: 10 is a sequence resulting from replacement of the 14th A of SEQ ID NO: 3 by G and the 19th U of SEQ ID NO: 3 by C, and SEQ ID NO: 12 is a sequence resulting from replacement of the 17th A of SEQ ID NO: 3 by U.

The length of the aptamer of the present invention is not particularly limited, and can usually be about 10 to about 200 nucleotides and can be, for example, not less than about 20 nucleotides (e.g., not less than 25 nucleotides, not less than 30 nucleotides, not less than 31 nucleotides, not less than 32 nucleotides, not less than 33 nucleotides), preferably not less than 25 nucleotides, more preferably not less than 30 nucleotides, further preferably not less than 33 nucleotides. In addition, it can be, for example, not more than about 100 nucleotides, generally not more than about 80 nucleotides, preferably not more than about 70 nucleotides, more preferably not more than about 60 nucleotides, further preferably not more than about 50 nucleotides, further preferably not more than about 45 nucleotides (e.g., not more than 44 nucleotides, not more than 43 nucleotides, not more than 42 nucleotides, not more than 41 nucleotides, not more than 40 nucleotides). When the total number of nucleotides is smaller, chemical synthesis and mass-production will be easier, and there is a major advantage in terms of cost. It is also thought that chemical modification is easy, stability in the body is high, and toxicity is low.

Therefore, the length of the aptamer of the present invention may be generally about 10 to about 200 nucleotides, preferably 20 to 80 nucleotides, more preferably 25 to 60 nucleotides, further preferably 25 to 50 nucleotides, most preferably 30 to 45 nucleotides.

The aptamer of the present invention may be a conjugate selected from the group consisting of a conjugate of plural aptamers containing a nucleotide sequence represented by the above-mentioned formula (1) (aptamers (A)), a conjugate of plural aptamers containing a nucleotide sequence resulting from replacement, deletion, insertion, or addition of 1 or several nucleotides in the nucleotide sequence represented by the above-mentioned formula (1) (aptamers (B)), and a conjugate of 1 or plural aptamers (A) and 1 or plural aptamers (B). These conjugates can also bind to FGF2.

Here, conjugation can be achieved by tandem binding. In the conjugation, a linker may be utilized. As the linker, nucleotide chains (e.g., 1 to about 20 nucleotides) and non-nucleotide chains (e.g., $-(CH_2)_n-$ linker, $-(CH_2CH_2O)_n-$ linker, hexaethylene glycol linker, TEG linker, peptide-containing linker, -S-S- bond-containing linker, -CONH- bond-containing linker, $-OPO_3-$ bond-containing linker) can be mentioned. The plurality in the above-described plural conjugates is not particularly limited as long as it is two or more, and it may be, for example, 2, 3, or 4.

Each nucleotide contained in the aptamer of the present invention is the same or different and can be a nucleotide containing a hydroxyl group at the 2' position of ribose (e.g., ribose of pyrimidine nucleotide, ribose of purine nucleotide) (i.e., a natural nucleotide) or a nucleotide wherein a hydroxyl group is replaced (modified) by any atom or group at the 2' position of ribose (sometimes to be indicated as "modified nucleotide" in the present invention).

As examples of any such atom or group, a nucleotide substituted by a hydrogen atom, a fluorine atom or an -O-alkyl group (e.g., -O-Me group), an -O-acyl group (e.g., -O-CHO group), or an amino group (e.g., $-NH_2$ group) can be mentioned. In the aptamer of the present invention, at least one kind (e.g., 1, 2, 3 or 4 kinds) of nucleotide can also be a modified nucleotide containing a hydroxyl group, or the above-described any atom or group, for example, at least two kinds (e.g., 2, 3 or 4 kinds) of groups selected from the group consisting of a hydrogen atom, a fluorine atom, a hydroxyl group and a -O-Me group, at the 2' position of ribose.

In the aptamer of the present invention, all pyrimidine nucleotides may be nucleotides wherein the 2'-position of ribose is a fluorine atom, or nucleotides wherein the fluorine atoms are not replaced, or replaced by any atom or group mentioned above, preferably an atom or group selected from the group consisting of a hydrogen atom, a hydroxyl group, and a methoxy group, though the manners of replacement and non-replacement are not bound. Particularly, when a production method using the DuraScribe™ T7 Transcription Kit (manufactured by Epicentre) is applied as a production method of the aptamer of the present invention, an aptamer wherein the 2'-position of ribose of all pyrimidine nucleotides is fluorinated can be obtained. The aptamer wherein a fluorine atom is replaced by other above-mentioned atom or group can be produced by the below-mentioned method.

In the aptamer of the present invention, all purine nucleotides may be nucleotides wherein the 2'-position of ribose is a hydroxy group, or nucleotides wherein the hydroxy groups are not replaced, or replaced by any atom or group mentioned above, preferably an atom or group selected from the group consisting of a hydrogen atom, a methoxy group, and a fluorine atom, though the manners of replacement and non-replacement are not bound. The aptamer wherein a hydroxyl group is replaced by other above-mentioned atom or group can be produced by the below-mentioned method.

In the aptamer of the present invention, moreover, all pyrimidine nucleotides may be nucleotides wherein the fluorine atom at the 2'-position of ribose is replaced by any of the aforementioned atoms or groups, for example, the same atoms or groups selected from the group consisting of a hydrogen atom, a hydroxy group, and an -O-Me group.

In the aptamer of the present invention, moreover, all purine nucleotides may be nucleotides wherein the hydroxy group at the 2'-position of ribose is replaced by any of the aforementioned atoms or groups, for example, the same atoms or groups selected from the group consisting of a hydrogen atom, a fluorine atom and an -O-Me group.

In a preferred embodiment, each pyrimidine nucleotide contained in the aptamer used in the present invention is a nucleotide having a fluorine atom at the 2'-position of ribose, and each purine nucleotide is a nucleotide having a hydroxy group at the 2'-position of ribose. In another embodiment, the above-mentioned fluorine atom at the 2'-position of the ribose of each pyrimidine nucleotide is independently optionally replaced by an atom or group selected from the group consisting of a hydrogen atom, a hydroxy group and a methoxy group, and the above-mentioned hydroxy group at the 2'-position of the ribose of each purine nucleotide is optionally independently replaced by an atom or group selected from the group consisting of a hydrogen atom, a methoxy group and a fluorine atom.

In this Description, the nucleotides constituting the aptamer are assumed to be RNAs (i.e., the sugar groups are assumed to be ribose) in describing how the sugar groups are modified in the nucleotides. However, this does not mean that DNA is exempted from the aptamer-constituting nucleotides, and a modification of RNA should read as a modification of DNA as appropriate. When the nucleotide constituting the aptamer is DNA, for example, replacement of the hydroxyl group at the 2'-position of ribose with other atom or functional group should read as a replacement of a hydrogen atom at the 2'-position of deoxyribose with such other atom or functional group.

When uracil is replaced by thymine in the aptamer of the present invention, FGF2-binding activity, FGF2-FGF receptor binding inhibitory activity, stability, drug deliverability and stability in blood of the aptamer and the like can be increased.

In the aptamer of the present invention, one or several, for example, 1 to 2, 1 to 3, 1 to 4, 1 to 5 nucleotides of phosphoric acid diester bond in the nucleotide may be modified or substituted by any substituent(s). For example, phosphoric acid diester bond may be replaced by a phosphorothioate bond, a phosphorodithioate bond, an alkylphosphonate bond, a phosphoramidate bond, and the like. Here, for example, "nucleotide is substituted by a phosphorothioate bond" means that a phosphoric acid group at a binding site between adjacent nucleotides is sulfurated, that is, a phosphodiester bond is altered to a phosphorothioate bond.

In the aptamer of the present invention, one or several, for example, 1 to 2, 1 to 3, 1 to 4, 1 to 5 nucleotides may be substituted by Bridged Nucleic Acid (BNA) or Locked Nucleic Acid (LNA) to stabilize the aptamer and improve the activity thereof. As used herein, the "bridged nucleic acid" refers to one having a structure wherein the binding affinity to a complementary sequence is enhanced by restricting the degree of freedom of nucleic acid by intramolecular crosslinking, and acquire nuclease resistance. Examples thereof include, but are not limited to, 2',4'-BNA (LNA), 2'-O,4'-C-ethylene-bridged Nucleic Acid (ENA), and the like.

The aptamer of the present invention may be one wherein a sugar residue (e.g., ribose) of each nucleotide has been further modified to increase the FGF2 binding activity, stability, drug deliverability, and the like. As examples of the modification in a sugar residue, replacement of oxygen atom at the 3'-position and/or the 4'-position of the sugar residue with another atom, and the like can be mentioned. As the kind of the modification, for example, fluorination, O-alkylation (e.g., O-methylation, O-ethylation), O-arylation, S-alkylation (e.g., S-methylation, S-ethylation), S-arylation, and amination (e.g., $-NH_2$) can be mentioned. It is possible to produce various variations of aptamers having enhanced activity even though the base sequence is the same, by further applying such alteration in the sugar residue to the aptamer of the present invention.

The aptamer of the present invention may also have a nucleic acid base (e.g., purine or pyrimidine) altered (e.g., chemically substituted) to increase the FGF2 binding activity, multimerization prevention, stability, drug deliverability, and the like. As examples of such alterations, pyrimidine alteration at 5-position, purine alteration at 6- and/or 8-position(s), alteration with an extracyclic amine, substitution with 4-thiouridine, and substitution with 5-bromo or 5-iodo-uracil can be mentioned. The phosphate group contained in the aptamer of the present invention may be altered to confer resistance to nuclease and hydrolysis. For example, the P(O)O group may be replaced by P(O)S (thioate), P(S)S (dithioate), P(O)N(R)R' (amidate), P(O)R, P(O)OR, CO or $CH_2$ (formacetal) or 3'-amine ($-NH-CH_2-CH_2-$) [wherein each R or R' is independently H or a substituted or unsubstituted alkyl (e.g., methyl, ethyl)].

The linking group is, for example, -O-, -N- or -S-, and nucleotides can bind to an adjoining nucleotide via these linking groups.

The alterations may also include alterations such as capping at 3' and 5'.

An alteration can further be performed by adding to an end a polyethylene glycol (PEG), amino acid, peptide, inverted dT, nucleic acid, nucleosides, Myristoyl, Lithocolic-oleyl, Docosanyl, Lauroyl, Stearoyl, Palmitoyl, Oleoyl, Linoleoyl, other lipids, steroids, cholesterol, caffeine, vitamins, dyes, fluorescent substances, anticancer agents, toxins, enzymes, radioactive substances, biotin, and the like. For such alterations, for example, US Patents 5,660,985 and 5,756,703 can be referred to.

Particularly, when alteration is performed by adding PEG to an end, the molecular weight of PEG is not particularly limited, and is preferably 1,000 to 100,000, more preferably 30,000 to 90,000. PEG may be linear or branched into two or more chains (multi-arm PEG). Addition of PEG to an end is useful for preventing the below-mentioned multimerization of the aptamer.

Such PEG is not particularly limited, and those of ordinary skill in the art can appropriately select and use commercially available or known PEG. Specific preferable examples of the PEG to be applied to the aptamer of the present invention include 2-branched GS type PEG having a molecular weight of 40,000 (SUNBRIGHT GL2-400GS manufactured by NOF CORPORATION), 2-branched TS type PEG having a molecular weight of 40,000 (SUNBRIGHT GL2-400TS manufactured by NOF CORPORATION), 4-branched TS type PEG having a molecular weight of 40,000 (SUNBRIGHT GL4-400TS manufactured by NOF CORPORATION), 2-branched TS type PEG having a molecular weight of 80,000 (SUNBRIGHT GL2-800TS manufactured by NOF CORPORATION), 4-branched TS type PEG having a molecular weight of 80,000 (SUNBRIGHT GL4-800TS manufactured by NOF CORPORATION), and the like.

In this case, in the aptamer of the present invention, PEG may be directly added to the terminus. It is more preferable that a linker having a group bindable to PEG and the like be added to the terminus thereof, and PEG be added to the aptamer of the present invention via the linker.

The linker for PEG and the aptamer of the present invention is not particularly limited, and carbon chain number, functional group, and the like can be appropriately selected according to the binding site, the kind of PEG, and the like. Examples of such linker include a linker having an amino group. Specifically, when added to the 5' end, ssH Linker (SAFC) or DMS(O)MT-AMINO-MODIFIER (GLEN RESEARCH) can be mentioned, and when added to the 3' end, TFA Amino C-6 lcaa CPG (ChemGenes), and the like can be mentioned. When this linker is selected, for example, an active group of N-hydroxysuccinimide is added to PEG, and reacted with an amino group on the linker side, whereby the aptamer of the present invention can be bound to PEG via the linker.

As PEG and linker, commercially available products can be preferably used. The reaction conditions and the like relating to the binding of PEG, a linker and the aptamer of the present invention can be appropriately determined by those of ordinary skill in the art.

More preferred embodiments of the aptamer of the present invention include the following aptamers.

aptamer ID1 containing the nucleotide sequence shown in SEQ ID NO: 3:

```
GL2-400TS-C6-
G(M)G(M)G(M)A(M)U(M)A(M)C(M)U(F)A(M)G(M)G(M)GC(M)A(M)U(M)U(F)A(
M)A(M)U(M)G(M)U(F)U(M)A(M)C(M)C(M)A(M)GU(F)GU(F)A(M)G(M)U(M)C(M
)C(M)C(M)-idT;
```

aptamer ID2 containing the nucleotide sequence shown in SEQ ID NO: 8:

```
GL2-400TS-C6-
C(M)G(M)G(M)A(M)U(M)A(M)C(M)U(F)A(M)G(M)G(M)GC(M)A(M)U(M)U(F)A(
M)A(M)U(M)G(M)U(F)U(M)A(M)C(M)C(M)A(M)GU(F)GU(F)A(M)G(M)U(M)C(M
)C(M)G(M)-idT;
```

aptamer ID3 containing the nucleotide sequence shown in SEQ ID NO: 9:

```
GL2-400TS-C6-

C(M)C(M)G(M)A(M)U(M)A(M)C(M)U(F)A(M)G(M)G(M)GC(M)A(M)U(M)U(F)A(

M)A(M)U(M)G(M)U(F)U(M)A(M)C(M)C(M)A(M)GU(F)GU(F)A(M)G(M)U(M)C(M

)G(M)G(M)-idT;
```

aptamer ID4 containing the nucleotide sequence shown in SEQ ID NO: 10:

```
GL2-400TS-C6-

G(M)G(M)G(M)A(M)U(M)A(M)C(M)U(F)A(M)G(M)G(M)GC(M)G(M)U(M)U(F)A(

M)A(M)C(M)G(M)U(F)U(M)A(M)C(M)C(M)A(M)GU(F)GU(F)A(M)G(M)U(M)C(M

)C(M)C(M)-idT;
```

aptamer ID5 containing the nucleotide sequence shown in SEQ ID NO: 12:

```
GL2-400TS-C6-

G(M)G(M)G(M)A(M)U(M)A(M)C(M)U(F)A(M)G(M)G(M)GC(M)A(M)U(M)U(F)U(

M)A(M)U(M)G(M)U(F)U(M)A(M)C(M)C(M)A(M)GU(F)GU(F)A(M)G(M)U(M)C(M

)C(M)C(M)-idT;  and
```

aptamer ID6 containing the nucleotide sequence shown in SEQ ID NO: 3:

```
idT-

G(M)G(M)G(M)A(M)U(M)A(M)C(M)U(F)A(M)G(M)G(M)GC(M)A(M)U(M)U(F)A(

M)A(M)U(M)G(M)U(F)U(M)A(M)C(M)C(M)A(M)GU(F)GU(F)A(M)G(M)U(M)C(M

)C(M)C(M)-C6-GL2-400TS
```

(in each of the above-mentioned formulas, (M) is a methoxy group at the 2'-position of ribose in each nucleotide, (F) is a fluorine atom at the 2'-position of ribose in each nucleotide, GL2-400TS is 2-branched TS type polyethylene glycol having a molecular weight of 40000, C6 is a -$(CH_2)_6$- linker, and idT is an inverted dT.)

Regardless of which of these aptamers is used, they can be stably present in the constitution of the therapeutic agent of the present invention, and are therefore suitable as the active ingredient of the therapeutic agent of the present invention.

The aptamer of the present invention may be a free form, or a pharmaceutically acceptable salt thereof. As such salt, a metal salt, an ammonium salt, an organic amine addition salt, an amino acid addition salt, and the like can be mentioned. As the metal salt, alkali metal salts such as sodium salt, potassium salt, and the like, alkaline earth metal salts such as magnesium salt, calcium salt, and the like, aluminum salt, zinc salt, and the like can be mentioned. As the ammonium salt, salts of ammonium, tetramethylammonium, and the like can be mentioned. As the organic amine addition salt, salts of tris hydroxyaminomethane and the like can be mentioned. As the amino acid addition salt, salts of lysine, arginine, histidine, tryptophan, ornithine, and the like can be mentioned.

The concentration of the aptamer of the present invention is not particularly limited, and any amount thereof may be contained as long as the therapeutic agent of the present invention affords the desired efficacy and effect. In the present specification, "the concentration of the aptamer" means the ratio (mg/mL) of the weight of the nucleic acid portion linked by the 5' →3' phosphoric acid bond constituting the aptamer molecule to the total volume of the therapeutic agent. As the concentration of the aptamer, for example, 1 to 60 mg/mL can be mentioned.

The aptamer of the present invention when present as a monomer binds to FGF2, and inhibits the function of FGF2 and exerts the efficacy thereof. However, when it is multimerized, the binding to FGF2 is not observed. It is thus considered that the aptamer does not inhibit the function of FGF2. When the aptamer concentration of the therapeutic agent of the present invention exceeds 20 mg/mL, the aptamer is multimerized even at the general storage temperature of 4 to 5°C, as a result of which the binding activity to FGF2 tends to decrease as a whole. Therefore, the upper limit of the concentration of the aptamer of the present invention in the therapeutic agent of the present invention is desirably a concentration not exceeding 20 mg/mL. When the concentration of the aptamer in the therapeutic agent of the present invention is low, the effect as a therapeutic agent may not be obtained. Therefore, while the lower limit of the concentration of the aptamer of the present invention in the therapeutic agent of the present invention is not particularly limited as long as the effect as a therapeutic agent can be obtained, it is desirably, for example, a concentration of not less than 1 mg/mL.

The dosage form of the therapeutic agent of the present invention is not particularly limited. It is preferably an aqueous liquid, more preferably an injection. When the therapeutic agent of the present invention is an aqueous liquid, the aptamer of the present invention, which is the active ingredient, is dissolved in a solvent.

As shown in WO 2020/004607, when PBS or physiological saline is used as the solvent, the aptamer of the present invention tends to lose stability and form a multimer due to the influence of the inorganic salt which is a strong electrolyte and, as a result, loses the binding activity to FGF2. On the other hand, water free of an inorganic salt (electrolyte) as a solvent suppresses multimerization of the aptamer of the present invention, and increases the proportion of the aptamer of the present invention present as a monomer. Furthermore, when the aptamer of the present invention dissolved in water is administered into the body, the higher-order structure required for aptamer activity is reconstructed by the action of the electrolyte present in the body fluid, and the activity can be exhibited. Therefore, the solvent used for the therapeutic agent of the present invention is preferably an aqueous solvent free of an inorganic salt (electrolyte), and water is particularly preferred.

When the therapeutic agent of the present invention is used as an injection, since a solvent free of an inorganic salt (electrolyte) is used as described above, the osmotic pressure is low and the agent cannot be used as an injection as it is. Therefore, the therapeutic agent of the present invention preferably contains a non-electrolyte osmoregulator (osmolyte) for the purpose of adjusting the osmotic pressure ratio to the plasma osmotic pressure to 1 or more, preferably 1 to 3.

The osmoregulator used for the therapeutic agent of the present invention is not particularly limited as long as it is a non-electrolyte, and may be any osmoregulator generally used other than inorganic ions (potassium ion, chloride ion, etc.). Examples of the osmoregulator include polyhydric alcohols (glycerol, mannitol, trehalose, glucose, sucrose, sorbitol, inositol, etc.), amino acids (alanine, glycine, glutamic acid, proline, GABA, taurine, ectin, etc.), methylammoniums (TMAO, choline, acetylcholine, glycine betaine, GPC, DMSP, etc.), ureas, and the like. Polyhydric alcohols are preferably used, and mannitol is more preferably used.

The amount of the osmoregulator is not particularly limited, and those skilled in the art can appropriately change the amount of the aptamer of the present invention contained in the therapeutic agent of the present invention according to the kind (molecular weight) of the osmoregulator to be used and the target osmotic pressure. For example, when mannitol is used as the osmoregulator and the ratio of the osmotic pressure to the physiological saline is about 1, the blending ratio of the osmoregulator in the entire injection is 2 to 7.5%(w/v). More specifically, when the ratio of the osmotic pressure to the physiological saline is 1, the blending ratio of mannitol is 4.9% when the concentration of the above-mentioned aptamer of the present invention is 2 mg/ml, and the blending ratio of mannitol is 3.6% when the concentration of the above-mentioned aptamer of the present invention is 20 mg/ml.

The therapeutic agent of the present invention is preferably substantially free of an electrolyte other than the aptamer of the present invention or a salt thereof to be the active ingredient. As used herein, "substantially free" means that a small amount of electrolyte may be contained as long as the FGF2 aptamer in the therapeutic agent of the present invention can be stably stored for a long term. More preferably, the therapeutic agent of the present invention does not contain an electrolyte other than the aptamer of the present invention or a salt thereof to be the active ingredient.

The therapeutic agent of the present invention may further contain a pharmaceutically acceptable additive where necessary. Examples of such additive include stabilizer, preservative, solubilizer, buffer, pH adjuster, soothing agent, and the like.

The pH of the therapeutic agent of the present invention is not particularly limited. When the agent is used as an injection, the pH is desirably near neutral, and can be appropriately selected within the range of, for example, 5 to 9, preferably 6 to 8.

The therapeutic agent of the present invention may contain other active ingredients as long as they do not adversely affect the activity and stability of the aptamer of the present invention. As such active ingredient, VGEF inhibitors such as McGen (registered trade mark), Lucentis (registered trade mark), Eylea (registered trade mark), Avastin (registered trade mark), and the like as therapeutic drugs for diseases accompanied by angiogenesis and the like, antiinflammatory agents such as steroids and the like, and analgesic/sedative agents such as morphine may be contained.

The therapeutic agent of the present invention obtained as described above can be used as a therapeutic agent for subretinal hyperreflective material or retinal diseases accompanying subretinal hyperreflective material.

Subretinal hyperreflective material (SHRM) is a hill-like structure formed from the retinal pigment epithelium toward the retina in the macula of retinal disease patients. Subretinal hyperreflective material can be detected by a known means or device, such as optical coherence tomography (OCT) or devices using such method. In the present specification, the subretinal hyperreflective material is a structure including neovascular tissue, fibrosis, subretinal hyperreflective exudation (SHE), and hemorrhage.

Such retinal diseases accompanying SHRM are not particularly limited as long as they are accompanied by SHRM. Examples thereof include vitreo-retinal lymphoma; BEST disease; retinal vitreous interface syndrome; central serous chorioretinopathy; myotonic dystrophy; age-related macular degeneration, and the like.

The therapeutic agent of the present invention can dramatically improve the therapeutic effect of the treatment of retinal diseases accompanying SHRM, by appropriately removing SHRM, which is a poor prognosis factor. Moreover, the agent can dramatically improve the prognosis thereof.

The age-related macular degeneration is not particularly limited as long as it is accompanied by subretinal hyperreflective material, and exudative age-related macular degeneration is preferred in terms of efficacy.

The subject of administration of the therapeutic agent of the present invention may be any mammal, and is a mammal that has developed subretinal hyperreflective material or a retinal disease accompanying subretinal hyperreflective material, or a mammal that may develop subretinal hyperreflective material or a retinal disease accompanying subretinal hyperreflective material. Examples of the mammal include experiment animals such as rodents (e.g., mouse, rat, hamster, guinea pig, and the like), and rabbit and the like, pets such as dog and cat and the like, domestic animals such as bovine, swine, goat, horse and sheep, and the like, primates such as human, monkey, orangutan and chimpanzee, and the like, and the like, and human is particularly preferred. The subject of administration may or may not be treated for subretinal hyperreflective material or a retinal disease accompanying subretinal hyperreflective material.

The therapeutic agent of the present invention can be administered parenterally (e.g., intravenous administration, intravitreally administration, instillation administration, and the like). The dosage of the therapeutic agent of the present invention varies depending on the structure of the aptamer of the present invention, severity of subretinal hyperreflective material or retinal diseases accompanying subretinal hyperreflective material, body weight, age, and the like of the subject of administration. The usual dosage, based on the amount of active ingredient (oligonucleotide site of aptamer) per day for an adult, can be about 0.0001 to about 100 mg/kg, for example, about 0.0001 to about 10 mg/kg, preferably about 0.005 to about 1 mg/kg.

[Example]

The present invention is explained in more detail in the following by referring to Examples; however, the present invention is not limited by the Examples.

Example 1: SHRM elimination effect of aptamer that binds to FGF2

In a phase I clinical study conducted to test the safety of RBM-007 (aptamer ID1 in the present specification) in the treatment of human eye disease (age-related macular degeneration: AMD), the inventors evaluated the tissue of the retina after administration of RBM-007, using an optical coherence tomography (OCT) scan.

RBM-007 was administered at a dose of 100 uL using a syringe with a 30-gauge needle into the vitreous body of the affected eye of an 86-year-old female patient suffering from age-related macular degeneration accompanying subretinal hyperreflective material (SHRM).

Then, the retina of the patient was evaluated by optical coherence tomography (OCT) scan. OCT is a technique that utilizes the relationship between light reflection/absorption to create high resolution images from the complicated tissues of the retinal layer. OCT was performed under the following conditions.

```
Device = Spectralis Diagnostic Imaging Platform - Heidelberg
Engineering, Inc. (spectral domain optical coherence tomography
device)
```

Settings: Imaging Mode: IR+OCT
Intensity IR: 100%
IR Field of View: 30°
Scan Pattern: Volume
Resolution: Hight Resolution (HR)
Acquisition Mode: Retina
EDI: ON
OCT ART Mean: 16 Frames

Scan Angle: 0° (horizontal)
Scan Pattern: Volume Scan (20°× 20°)
# B-Scan Sections: 49 lines (dense scan)
Scan Quality (Q): ≥20

An OCT image of the right eye of an 86-year-old female patient on day 0 of administration is shown in Fig. 1 (upper panel). In the evaluation of a test subject by OCT, the inventors identified a typical structure associated with neovascular tissue, fibrosis, and subretinal hyperreflective exudation (SHE) and confirmed hemorrhage. This structure is called subretinal hyperreflective material (SHRM) and is a tissue found in many retinal diseases. In particular, SHAM caused by exudative type age-related macular degeneration is considered to be one of the causes of poor prognosis.

An OCT image of the right eye of the 86-year-old female patient on the 56th day of administration is shown in Fig. 1 (lower panel). As a result, it was found that SHRM was completely reabsorbed and disappeared on the 56th day after the single administration of RBM-007. It has been proved that the persistence of SHRM is involved in fibrosis, atrophia, and decreased eyesight in patients with age-related macular degeneration, and the disappearance of SHRM observed in this Example shows that RBM-007 of the present invention is extremely effective in treating age-related macular degeneration accompanying SHRM formation. Moreover, since RBM-007 was shown to have an effect of eliminating SHRM, the aptamer of the present invention prevents fiber formation in not only AMD but also a large number of retinal diseases, and good therapeutic results can be expected. The inventors expect this effect in phase II clinical test.

[Industrial Applicability]

The present invention can provide an effective therapeutic agent for subretinal hyperreflective material, and further, retinal diseases accompanying subretinal hyperreflective material, for which a therapeutic drug has not existed so far, and improvement of the prognosis of retinal diseases accompanying subretinal hyperreflective material can be expected. This application is based on a patent application No. US 62/970,842 filed in the US (filing date: February 6, 2020), the contents of which are incorporated in full herein.

**Claims**

1.    A therapeutic agent for subretinal hyperreflective material or a retinal disease accompanying subretinal hyperreflective material, comprising an aptamer that binds to FGF2 or a salt thereof.

2.    The therapeutic agent according to claim 1, wherein the aptamer is an aptamer comprising a nucleotide sequence represented by the following formula (1)

$$N^1GGAN^2ACUAGGGCN^3UUAAN^4GUN^5ACCAGUGUN^6 \qquad (1)$$

wherein $N^1$ and $N^6$ are each independently any 0 to several bases, and $N^2$, $N^3$, $N^4$ and $N^5$ are independently any one base, provided that uracil is optionally thymine: and is the following (a) or (b):

(a) an aptamer wherein, in the nucleotides contained in the aptamer,

(i) the 2'-position of the ribose of each pyrimidine nucleotide is a fluorine atom,
(ii) the 2'-position of the ribose of each purine nucleotide is a hydroxy group;

(b) the aptamer of (a), wherein

(i) the fluorine atom at the 2'-position of the ribose of each pyrimidine nucleotide is independently not replaced, or replaced by an atom or group selected from the group consisting of a hydrogen atom, a hydroxy group, and a methoxy group,
(ii) the hydroxy group at the 2'-position of the ribose of each purine nucleotide is independently not replaced, or replaced by an atom or group selected from the group consisting of a hydrogen atom, a methoxy group, and a fluorine atom.

3.    The therapeutic agent according to claim 2, wherein the nucleotide sequence represented by the formula (1) is a nucleotide sequence represented by the following formula (3):

$N^1$GGAUACUAGGGCAUUAAUGUUACCAGUGUAGUC$N^{62}$ (3)

wherein $N^1$ and $N^{62}$ are each independently any 0 to several bases.

4.  The therapeutic agent according to claim 3, wherein the nucleotide sequence represented by the formula (3) is a nucleotide sequence shown in any of SEQ ID NO: 1, 3, 4, 5, 6, or 8.

5.  The therapeutic agent according to claim 3, wherein the nucleotide sequence represented by the formula (3) is a nucleotide sequence shown in SEQ ID NO: 3.

6.  The therapeutic agent according to any one of claims 2 to 5, wherein the aptamer is an aptamer represented by the following:

```
GL2-400TS-C6-
G(M)G(M)G(M)A(M)U(M)A(M)C(M)U(F)A(M)G(M)G(M)GC(M)A(M)U(M)U(F)A(
M)A(M)U(M)G(M)U(F)U(M)A(M)C(M)C(M)A(M)GU(F)GU(F)A(M)G(M)U(M)C(M
)C(M)C(M)-idT
```

wherein (M) is a methoxy group at the 2'-position of ribose in each nucleotide, (F) is a fluorine atom at the 2'-position of ribose in each nucleotide, GL2-400TS is a 2-branched TS type polyethylene glycol having a molecular weight of 40000, C6 is a -$(CH_2)_6$- linker, and idT is an inverted dT.

7.  The therapeutic agent according to any one of claims 1 to 6, wherein the retinal disease is age-related macular degeneration.

8.  The therapeutic agent according to any one of claims 1 to 7, wherein the agent is administered in combination with an anti-VEGF drug.

9.  A method for treating subretinal hyperreflective material or a retinal disease accompanying subretinal hyperreflective material, comprising administering an effective amount of an aptamer that binds to FGF2 or a salt thereof to a patient thereof.

10. An aptamer that binds to FGF2 or a salt thereof for treating subretinal hyperreflective material or a retinal disease accompanying subretinal hyperreflective material.

11. Use of an aptamer that binds to FGF2 or a salt thereof for the production of a therapeutic agent for subretinal hyperreflective material or a retinal disease accompanying subretinal hyperreflective material.

[Fig. 1]

**INTERNATIONAL SEARCH REPORT**

International application No.

PCT/JP2021/004215

**A. CLASSIFICATION OF SUBJECT MATTER**
A61K 31/7088(2006.01)i; A61K 45/00(2006.01)i; A61P 27/02(2006.01)i; C12N
15/115(2010.01)i
FI: A61K31/7088 ZNA; A61K45/00; A61P27/02; C12N15/115 Z

According to International Patent Classification (IPC) or to both national classification and IPC

**B. FIELDS SEARCHED**

Minimum documentation searched (classification system followed by classification symbols)
A61K31/7088; A61K45/00; A61P27/02; C12N15/115

Documentation searched other than minimum documentation to the extent that such documents are included in the fields searched
Published examined utility model applications of Japan 1922–1996
Published unexamined utility model applications of Japan 1971–2021
Registered utility model specifications of Japan 1996–2021
Published registered utility model applications of Japan 1994–2021

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS
(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2020/004607 A1 (RIBOMIC INC.) 02 January 2020 (2020-01-02) paragraphs [0014]–[0042] | 10<br>1–11 |
| X<br>Y | MATSUDA, Y. et al., "Anti-angiogenic and anti-scarring dual action of an anti-fibroblast growth factor 2 aptamer in animal models of retinal disease", Mol. Ther. Nucleic Acids, 2019, vol. 17, pp. 819–828, pp. 820, 823, 824, fig. 4–6 | 1–11<br>1–11 |
| Y | JP 2009-509962 A (INTERMUNE, INC.) 12 March 2009 (2009-03-12) paragraphs [0003], [0004], [0049], [0052], [0054] | 1–11 |

☒ Further documents are listed in the continuation of Box C.   ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

Date of the actual completion of the international search
10 March 2021 (10.03.2021)

Date of mailing of the international search report
23 March 2021 (23.03.2021)

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

| | International application No. |
|---|---|
| | PCT/JP2021/004215 |

C (Continuation).   DOCUMENTS CONSIDERED TO BE RELEVANT

| Category* | Citation of document, with indication, where appropriate, of the relevant passages | Relevant to claim No. |
|---|---|---|
| A | 木村和博，難治性網膜硝子体疾患における線維性増殖織形成に対する Sex hormone の作用，山口医学, 2015, vol. 64, pp. 11-16, entire text, (KIMURA, Kazuhiro, "Action of Sex Hormone for Proliferative Fibrocellular Tissue Formation in Refractory Vitreoretinal Diseases", Yamaguchi Medical Journal) | 1-11 |
| A | JP 2020-500938 A (BORODIC, Gary E.) 16 January 2020 (2020-01-16) paragraph [0093], example 4 | 1-11 |

Form PCT/ISA/210 (continuation of second sheet) (January 2015)

INTERNATIONAL SEARCH REPORT
Information on patent family members

International application No.

PCT/JP2021/004215

| Patent Documents referred in the Report | Publication Date | Patent Family | Publication Date |
|---|---|---|---|
| WO 2020/004607 A1 | 02 Jan. 2020 | (Family: none) | |
| JP 2009-509962 A | 12 Mar. 2009 | US 2009/0191265 A1 paragraphs [0005], [0006], [0061], [0064], [0066] WO 2007/038315 A2 EP 1940364 A2 KR 10-2008-0046673 A CN 101267810 A | |
| JP 2020-500938 A | 16 Jan. 2020 | US 2018/0296651 A1 paragraph [0133], example 4 WO 2018/107005 A1 EP 3551219 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020004607 A
- US 5660985 A
- US 5756703 A
- US 62970842

**Non-patent literature cited in the description**

- **BROWN DM et al.** *N Engl J Med.,* 05 October 2006, vol. 355 (14), 1432-44
- **BHISITKUL RB et al.** *Ophthalmology,* June 2016, vol. 123 (6), 1269-77
- **ALEX S ; WILLOUGHBY, BS et al.** *Graefes Arch Clin Exp Ophthalmol,* 2018, vol. 256, 2089-2096
- **EBENEZER DANIEL et al.** *Ophthalmology,* March 2014, vol. 121 (3), 656-666
- **CICINELLI, MARIA VITTORIA et al.** *Retin Cases Brief Rep.,* 2019
- **DANIELA C. FERRARA et al.** *Graefes Arch Clin Exp Ophthalmol.,* October 2010, vol. 248 (10), 1377-1386
- **SARRA GATTOUSSI et al.** *Acta Ophthalmol.,* 2019, vol. 97, 364-371
- **NIROJ K SAHOO et al.** *Indian Journal of Ophtalmology,* vol. 68 (1), 126-129
- **A OZKAYA et al.** *J Fr Ophtalmol.,* 2018, vol. 41, 21-29